# EUROPEAN PATENT SPECIFICATION

(11) **EP 3 925 592 B1**
(45) Date of publication and mention of the grant of the patent: **17.04.2024**
(21) Application number: 19914856.0
(22) Date of filing: 12.07.2019
(51) Int. Cl.: A61J 7/00, A61J 1/20

(54) **DEVICE FOR CRUSHING AND DISSOLVING/SUSPENDING DANGEROUS DRUGS FOR ENTERAL ADMINISTRATION**
VORRICHTUNG ZUM ZERKLEINERN UND AUFLÖSEN/SUSPENDIEREN VON GEFÄHRLICHEN MEDIKAMENTEN ZUR ENTERALEN VERABREICHUNG
DISPOSITIF POUR LA TRITURATION ET LA DISSOLUTION/SUSPENSION DE MÉDICAMENTS DANGEREUX À ADMINISTRATION PAR VOIE ENTÉRALE

(30) Priority: 11.02.2019 ES 201930216 U
(43) Date of publication of application: 22.12.2021
(73) Proprietor: Vidal Lopez, Carlos Francisco, 15001 La Coruña (ES)
(72) Inventor: Vidal Lopez, Carlos Francisco, 15001 La Coruña (ES)
(74) Representative: González López-Menchero, Álvaro Luis
(86) International application number: PCT/ES2019/070491
(87) International publication number: WO 2020/165469

(56) References cited:
- WO-A1-2005/025482
- JP-A- 2012 120 769
- US-A1- 2005 078 888
- US-A1- 2012 046 636
- US-A1- 2014 294 952
- US-A1- 2016 354 282
- DATABASE WPI Week 24706 Thomson Scientific, London, GB; AN 2015-24706W XP055730491, "Drug delivery kit for drug delivery system, has two hollow needle units that are penetrated and passed in fourth hollow needle unit to transfer chemical agent between injection syringe barrels", & WO 2015/049730 A1 (MEDICAL CREATION INC)

## Description

### OBJECT OF THE INVENTION

The invention, as stated in the title of the present specification, relates to a device for crushing and dissolving/suspending dangerous drugs for enteral administration, which provides advantages and features, described in detail below, which represent an improvement on the current state of the art, to the function for which it is intended.

More specifically, the object of the invention focuses on a device whose purpose is to provide a means of safe handling that avoids direct contact and contamination in the processes of crushing and dissolving/suspending in water of dangerous solid drugs for enteral administration, which is based on a system compatible with enteral administration syringes with ENFIt^{®} type connection, required by current regulations, which in an innovative and advantageous way is constituted as a closed system, that is to say, it does not require the disconnection of the syringe at any time during the process, avoiding any possibility of contact with the product.

### FIELD OF APPLICATION OF THE INVENTION

The field of application of the present invention falls within the sector of the medical device manufacturing industry, focusing particularly on the field of those applicable for the safe handling of drugs.

### BACKGROUND TO THE INVENTION

As is well known, there are a number of drugs, known as dangerous drugs, whose toxicity poses a severe risk to the handler. These drugs are carcinogenic, teratogenic drugs, with reproductive toxicity or organ toxicity at low doses.

In this regard, the National Institute for Safety and Hygiene at Work has published a technical document in which, for each of them, it establishes the appropriate recommendations for their handling.

This document recommends that these drugs should be handled using closed transfer systems that prevent exposure. These are connectors for handling parenterally administered drugs packaged in vials and dosed via syringes, which should be connected to these vials via the aforementioned systems.

There is an unresolved problem for dangerous drugs formulated in tablet form that need to be administered to patients with swallowing problems (due to age or various health problems). There is currently no safe device that allows the tablet to be crushed and then suspended/dissolved in water and finally conditioned in a syringe for enteral administration.

Said process generates a suspended dust of a toxic product that may pose an unacceptable risk of exposure for handlers (in the hospital or domestic setting) and of cross-contamination of other preparations in the hospital environment.

Moreover, current regulations (ISO 80369-3) require that syringes and accessory devices for the enteral administration of drugs have their own type of connection (ENFit^{®} connection), which is incompatible with parenteral administration syringes in order to avoid confusion over the routes of administration.

In addition to the fact that the aforementioned drug transfer systems currently available are not compatible with this type of syringe, and therefore need to be adapted, they are also not completely reliable, as they require at least one step of disconnecting and connecting the syringe to be performed, which entails the risk of contact with the product by the personnel handling the device.

Specifically, a system is known which, formed based on a pouch, is designed to introduce therein the dry tablet of the drug, to subsequently introduce the water or fluid to dissolve same through a syringe connected to the pouch and to then proceed to crush it in a pressure mechanism in which the pouch is introduced, finally being necessary to reabsorb the mixture with the syringe and disconnect it to then connect it to the enfit connector, suitable for enteral feeding.

Therefore, in addition to other disadvantages, the main problem with this system is the need to disconnect the syringe and then connect it to the enfit connector for enteral feeding, as during this disconnection phase it is very easy for the product to leak and come into contact with the professional who is handling it.

In addition, another problem with this system is the poor resistance to pressure of the sealing system of the opening of the bag provided for the introduction of the tablets inside it, since said opening, which must be wide enough to allow the introduction of the tablet, usually consists of an opening that covers the entire side of the bag and has a zip or zip-type sealing system, which has the disadvantage that when the bag is subjected to pressure from the mechanism, either in a crusher of the tablet to convert it into powder or to make the mixture that enables the dilution of said powder in the fluid, there is a double risk of leakage as said zip closure is not strong enough to withstand the force exerted on the pouch and tends to open, with the consequent risk of contamination.

In the state of the art is known the document JP2012120769, which discloses an administer for a suspended drug obtained by crushing a tablet in a closed system and suspending the crushed tablet in the closed system, to a patient in the closed system.

In conclusion, a device is needed which on the one hand allows the drug to be crushed and suspended/dissolved safely and which is also provided with openings with secure closures for the introduction of the tablets, but above all, with secure connections for the coupling thereof to the enteral administration syringe without the need to disconnect the same at any stage of the process for the connection thereof to the enteral feeding tube or, if the patient can swallow directly by mouth, to be able to do so without the need to disconnect the syringe, i.e. it is configured as a closed and safe system for carrying out the crushing and dissolving/suspension of dangerous drugs for enteral administration.

This solution would comply with the provisions of current legislation, which establishes the obligation to adopt measures to avoid or minimise exposure to dangerous drugs. The object of the present invention is, therefore, the development of a closed-circuit device that allows the aforementioned process of crushing and dissolving/suspending this type of drug to be carried out safely and, in addition, can be coupled to the ENFIt^{®} connections of the syringes for this type of enteral administration.

Furthermore, and as a reference to the current state of the art, it should be pointed out that the applicant at least is unaware of the existence of any other device, or any other invention of similar application, with technical, structural and constitutive characteristics that are the same or similar to those of the one claimed here.

### DESCRIPTION OF THE INVENTION

The invention is disclosed in the claims. The device for crushing and dissolving/suspending dangerous drugs for enteral administration proposed by the invention satisfactorily achieves the above-mentioned objectives, the characteristic details that make it possible and that distinguish it being conveniently included in the final claims accompanying the present description.

More specifically, the device that the invention proposes, as mentioned above, provides a safe handling means that avoids direct contact and contamination in the processes of crushing and dissolving/suspending in water of dangerous solid drugs for enteral administration, being based on a closed system compatible with enteral administration syringes with ENFIt^{®} type connection, required by current regulations. For this purpose, and more specifically, this device is essentially made up of two different parts: a crushing and dispersing area in water and another area for fitting to the enteral dosing syringe.

The crushing area thus comprises a mixing bag, preferably with a capacity of 100 ml, made of ethylene-vinyl acetate or polylefins. This bag has, on the one hand, an opening with an airtight, pressure-resistant seal, preferably a screw cap, which is used as the inlet for the tablet, and, on the other hand, a second opening, for the passage of fluid, equipped with a three-way valve, to which an ENFit^{®} type syringe for enteral administration and a tube at the distal end of which a connector for a probe is incorporated.

Thus, the handling of the device for crushing and dissolving/suspending the solid drug would be as follows:
Once the tablet is introduced into the mixing bag through the first inlet provided for this purpose with the screw cap, the bag is hermetically sealed and the syringe is connected to the three-way valve connection located in the second inlet of the bag, making the bag completely closed.

The tablet is then crushed, a process that will no longer produce any powder that can be spread outside the bag, not even by the pressure exerted, and then the valve is opened and, using the syringe, the necessary water is injected into the mixing bag to disperse the powder formed and, after the mixture has been properly stirred with the valve closed, the valve is opened again and the drug is loaded into the syringe by suctioning the same from the inside of the bag.

The valve is then closed again and the drug is injected into the patient through the delivery tube, either directly into the patient's mouth, when the patient is able to swallow the enteral feeding, by inserting the end of the tube with the connector into the patient's mouth, or by connecting said connector to the nasogastric tube for enteral feeding, without having to disconnect the syringe at any time to do so, making the system completely safe.

Furthermore, the described three-way valve that incorporates the device of the invention provides an added advantage and that is that, thanks to the fact that, depending on which way it is positioned, it closes the passage, playing with these options it is possible to do the following:
- fill the bag, if the outlet to the tube is closed,
- avoid return to the bag if the stopcock is positioned to close the outlet from the syringe after suctioning with the syringe.

This is important because the mixture can thus be transported inside the device from the preparation area to the patient site without risk of spillage or leakage.

In addition, this allows its use in various settings (pharmacy services where drug mixtures are prepared and sent to the nursing units) in the nursing units themselves at times when the pharmacy service is not available, or in the patient's own home to avoid risks due to the toxicity of the drugs.

In addition and complementarily, a second three-way valve could be connected to the tube connector and could be used to be able to clean the drug supply tubing coming out of the bag of any drug residue, and wherein a second tubing and a second connector is arranged on one of the outlets. This flushing carried out by means of the second three-way valve allows, on the one hand, the complete administration of the drug dose and, on the other hand, safety in the disconnection.

Both in the embodiment with a single three-way valve and in the embodiment with a second three-way valve, a bioconnector or connector with "enfit" threaded sections or a feeding tube can be connected.

The bioconnector, also known as a "needle-free connector", allows for safe disconnection.

Thanks to the features of the elements described, their functionality and the interoperability between them, it is possible to comply, among others, with the American USP-800 standard, which clearly states that examples of means of delivering dangerous drugs would be the crushing of tablets in plastic bags, and that the administration is carried out using closed systems.

### DESCRIPTION OF THE DRAWINGS

In order to complement the description being made and for the purpose of helping to make the features of the invention more readily understandable, this specification is accompanied, as an integral part thereof, by a drawing in which, by way of illustration and not limitation, represents the following:
Figure 1.- Shows a schematic perspective view of an example of the device for crushing and dissolving/suspending dangerous drugs for enteral administration object of the invention, showing the parts and elements it comprises, as well as the configuration and arrangement thereof.
Figure 2.- Shows a schematic representation in perspective that complements the previous embodiment by means of the connection of a second three-way valve.
Figure 3.- Shows a perspective representation of additional elements connectable to the connector of both the embodiment shown in figure 1 and figure 2.

### PREFERRED EMBODIMENT OF THE INVENTION

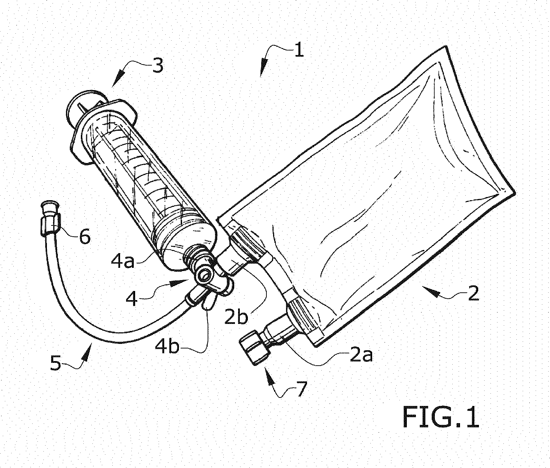

In view of the described figure 1 and only, and in accordance with the adopted numbering, it is possible to observe therein a non-limiting exemplary embodiment of the device of the invention, which comprises that which is indicated and described in detail below.

Thus, as can be seen in the said figure, the device (1) in question is of the type configured from a set of elements which, once coupled together, constitute a closed system for handling the dangerous solid drug, comprising, as is well known, a mixing bag (2), for the crushing and dispersing in water of the drug, which is equipped with a first opening (2a) suitable as an inlet for the insertion therein of the tablet which constitutes the drug and a second opening (2b) for the passage of fluid, and a syringe (3) with which water is injected to disperse the powder which the tablet has become once crushed inside the bag and with which the drug is suctioned once diluted and mixed, with the particular feature that said syringe (3) is an enteral syringe and is coupled to the second opening (2b) of the mixing bag (2) by interposing a three-way valve (4) which, hermetically connected to said opening (2b), has:
- an ENFIt-type connection (4a) to connect the enteral syringe (3),
- an extension tube (5) provided with a probe connector (6) at the distal end thereof,
- and a movable stopcock (4b) which, depending on the position it is located in, opens the passage of fluid from the syringe (3) to the opening (2b) of the mixing bag (2), to inject the solution water and to suction the mixture once the dissolution has been carried out, or from the syringe (3) to the tube (5) to deliver the diluted drug directly into the patient's mouth or by connecting the connector (6) to a nasogastric tube, making any disconnection of the enteral syringe (3) unnecessary during the whole process.

Furthermore, also in an innovative manner, the first opening (2a) of the mixing bag (2) is a circular opening equipped with a hermetic and pressure resistant screw seal (7), both openings (2a, 2b) being the only openings which the mixing bag (2) has, which, moreover, is totally hermetic and watertight, the entire perimeter thereof being sealed by means of high pressure resistant heat welding, preventing any leakage of contents during the crushing process to which it is subjected once the tablet for which it is intended has been introduced.

Preferably, the mixing bag (2) is made of ethylene vinyl acetate or polylefins and, also preferably, has a capacity of approximately 100 ml. Furthermore, both openings (2a, 2b) of the bag (2) can be arranged on the same side or on opposite sides of the same.

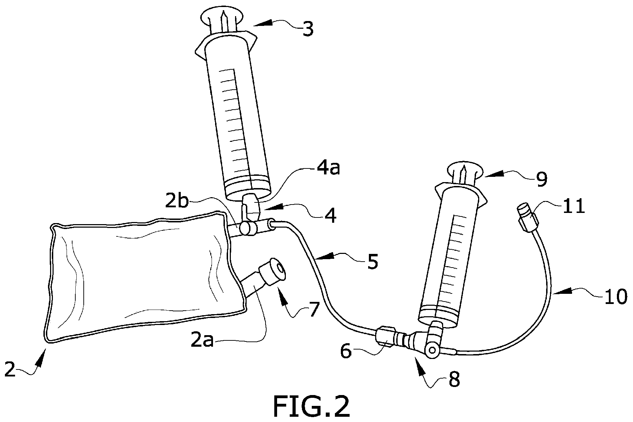

Figure 2 shows how a second three-way valve (8) is mounted on the connector (6) arranged at the end of the tubing (5), where a second syringe (9) is connectable to one of the inlets, while a second section of tubing (10) is connectable to the other of the outlets of this three-way valve (8), and at the end of which there is a connector (11).

The second syringe (9) connectable to the second three-way valve (8) is used to inject water towards the first section of tubing (5) and thus clean said tubing (5) of any remaining drug.

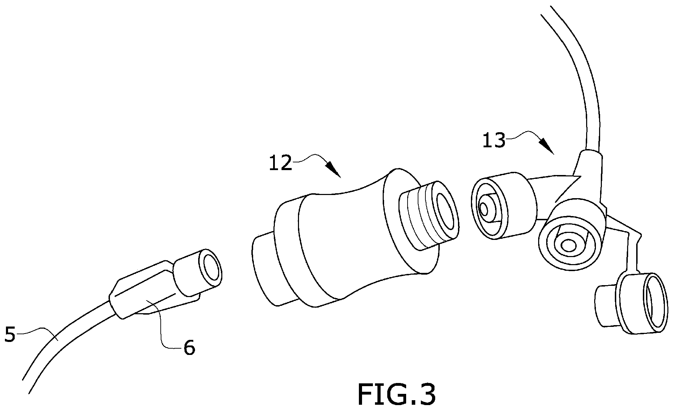

Figure 3 aims to show that either a bioconnector (12) provided with "enfit" threaded sections that are not compatible with intravenous catheters or feeding tubes (3) both on the connector (6) of the first section of tubing (5) of the embodiment shown in figure 1 and on the connector (11) of the second section of tubing (10) of the embodiment shown in figure 2 is connectable both on the connector (6) of the first section of tubing (5) of the embodiment shown in figure 1 and on the connector (11) of the second section of tubing (10) of the embodiment shown in figure 2.

## Claims

1. A DEVICE FOR CRUSHING AND DISSOLVING/SUSPENDING DANGEROUS DRUGS FOR ENTERAL ADMINISTRATION, comprising a mixing bag (2) , a syringe (3) with which water is injected to disperse the powder which the tablet has become once crushed inside the bag and with which the drug is suctioned once diluted and mixed and a three-way valve (4),
**characterised in that** the mixing bag (2) for crushing and dispersing the solid drug in water is provided with a first opening (2a) suitable as an inlet for the insertion therein of the tablet that constitutes the drug and a second opening (2b) for the passage of fluid and **in that** said syringe (3) is an enteral syringe and is coupled to the second opening (2b) of the mixing bag (2) by interposing the three-way valve (4) which, hermetically connected to said opening (2b), has: with an ENFIt-type connection (4a) for connecting the enteral syringe (3); with an extension tube (5) provided with a connector (6) for a tube at the distal end thereof; and with a movable stopcock (4b) which, depending on the position it is located in, opens the passage of fluid from the syringe (3) to the opening (2b) of the mixing bag (2), to inject the solution water and to suction the mixture once dissolution has been performed, or from the syringe (3) to the tube (5) to deliver the diluted drug directly into the patient's mouth or by connecting the connector (6) to a nasogastric tube, without the need to disconnect the enteral syringe (3).

2. THE DEVICE FOR CRUSHING AND DISSOLVING/SUSPENDING DANGEROUS DRUGS FOR ENTERAL ADMINISTRATION, according to claim 1, **wherein** the first opening (2a) of the mixing bag (2) is a circular opening equipped with a hermetic and pressure resistant screw seal (7).

3. THE DEVICE FOR CRUSHING AND DISSOLVING/SUSPENDING DANGEROUS DRUGS FOR ENTERAL ADMINISTRATION, according to claims 1 and 2, **wherein** both openings (2a, 2b) of the mixing bag (2) are the only openings that said mixing bag (2) has and it is totally hermetic and watertight, the entire perimeter thereof being sealed by means of high pressure resistant heat welding.

4. THE DEVICE FOR CRUSHING AND DISSOLVING/SUSPENDING DANGEROUS DRUGS FOR ENTERAL ADMINISTRATION, according to claim 3, **wherein** the mixing bag (2) is made of ethylene vinyl acetate or polylefin and has a capacity of approximately 100 ml.

5. THE DEVICE FOR CRUSHING AND DISSOLVING/SUSPENDING DANGEROUS DRUGS FOR ENTERAL ADMINISTRATION, according to claim 3 or 4, **wherein** both openings (2a, 2b) of the bag (2) are arranged on the same side thereof.

6. THE DEVICE FOR CRUSHING AND DISSOLVING/SUSPENDING DANGEROUS DRUGS FOR ENTERAL ADMINISTRATION, according to claim 3 or 4, **wherein** both openings (2a, 2b) of the bag (2) are on opposite sides thereof.

7. A DEVICE FOR CRUSHING AND DISSOLVING/SUSPENDING DANGEROUS DRUGS FOR ENTERAL ADMINISTRATION according to any of the preceding claims, in that a second three-way valve (8) is mounted on the connector (6) arranged at the end of the tube (5), wherein a second syringe (9) is connectable on one of the inlets, while a second section of tubing (10) is connectable on the other of the outlets of this three-way valve (8), and at the end of which there is a connector (11).

8. THE DEVICE FOR CRUSHING AND DISSOLVING/SUSPENDING DANGEROUS DRUGS FOR ENTERAL ADMINISTRATION on the connector (6) of the first section of tubing (5) or on the connector (11) of the second section of tubing (10) is connectable either a bioconnector (12) provided with "enfit" threaded sections which are not compatible with intravenous catheters, or nasogastric tubes (3).

## Patentansprüche

1. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG, die einen Mischbeutel (2), eine Spritze (3), mit der Wasser injiziert wird, um das Pulver, zu dem die Tablette nach dem Zerstoßen wurde, im Beutel zu dispergieren, und mit der das Medikament nach Verdünnen und Mischen angesaugt wird, sowie ein Dreiwegeventil (4) umfasst,
**dadurch gekennzeichnet, dass** der Mischbeutel (2) zum Zerstoßen und Dispergieren des festen Medikaments in Wasser mit einer ersten Öffnung (2a), die als ein Einlass für das Eingeben der das Medikament konstituierenden Tablette darein geeignet ist, und einer zweiten Öffnung (2b) zur Durchleitung eines Fluids versehen ist, und dadurch, dass die Spritze (3) eine enterale Spritze und durch Einfügen des Dreiwegeventils (4), das hermetisch mit der Öffnung (2b) verbunden ist und Folgendes aufweist, an die zweite Öffnung (2b) des Mischbeutels (2) gekoppelt ist: mit einer ENFIt-Verbindung (4a) zum Verbinden der enteralen Spritze (3); mit einem Verlängerungsschlauch (5), der am distalen Ende davon mit einem Verbinder (6) versehen ist; und mit einem bewegbaren Absperrhahn (4b), der in Abhängigkeit von der Position, in der er sich befindet, die Durchleitung eines Fluids von der Spritze (3) zur Öffnung (2b) des Mischbeutels (2), um das Lösungswasser zu injizieren und das Gemisch, nachdem eine Auflösung durchgeführt wurde, ansaugt, oder von der Spritze (3) zum Schlauch (5) öffnet, um das verdünnte Medikament direkt in den Mund des Patienten oder durch Verbinden des Verbinders (6) mit einem nasogastralen Schlauch zu geben, ohne dass die enterale Spritze (3) getrennt werden muss.

2. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG nach Anspruch 1, **wobei** die erste Öffnung (2a) des Mischbeutels (2) eine kreisförmige Öffnung ist, die mit einer hermetischen und druckbeständigen Schraubdichtung (7) ausgerüstet ist.

3. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG nach Anspruch 1 und 2, **wobei** beide Öffnungen (2a, 2b) des Mischbeutels (2) die einzigen Öffnungen sind, die der Mischbeutel (2) aufweist, und sie vollständig hermetisch und wasserdicht ist, wobei der gesamte Umfang davon mittels einer hochdruckbeständigen Wärmeschweißung abgedichtet ist.

4. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG nach Anspruch 3, **wobei** der Mischbeutel (2) aus Ethylenvinylacetat oder Polylefin besteht und eine Kapazität von ungefähr 100 ml aufweist.

5. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG nach Anspruch 3 oder 4, **wobei** beide Öffnungen (2a, 2b) des Beutels (2) auf derselben Seite davon angeordnet sind.

6. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG nach Anspruch 3 oder 4, **wobei** sich beide Öffnungen (2a, 2b) des Beutels (2) auf gegenüberliegenden Seiten davon befinden.

7. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG nach einem der vorhergehenden Ansprüche, dass ein zweites Dreiwegeventil (8) am Verbinder (6) montiert ist, der am Ende des Schlauches (5) angeordnet ist, wobei eine zweite Spritze (9) mit einem der Einlässe verbindbar ist, während ein zweiter Abschnitt eines Schlauches (10) nit dem anderen der Auslässe des Dreiwegeventils (8) verbindbar ist, an dessen Ende sich ein Verbinder (11) befindet.

8. VORRICHTUNG ZUM ZERSTOSSEN UND AUFLÖSEN/SUSPENDIEREN VON GEFÄHRLICHEN MEDIKAMENTEN FÜR EINE ENTERALE VERABREICHUNG am Verbinder (6) des ersten Abschnitts des Schlauches (5) oder am Verbinder (11) des zweiten Abschnitts des Schlauches (10) entweder einem Bioverbinder (12), der mit "Enfit"-Gewindeabschnitten versehen ist, die mit intravenösen Kathetern nicht kompatibel sind, oder nasogastralen Schläuchen (3) verbindbar ist.

## Revendications

1. DISPOSITIF DE CONCASSAGE ET DE DISSOLUTION/SUSPENSION DE MÉDICAMENTS DANGEREUX POUR ADMINISTRATION ENTÉRALE, comprenant un sachet de mélange (2), une seringue (3) avec laquelle de l'eau est injectée pour disperser la poudre qu'est devenu le comprimé une fois concassé à l'intérieur du sachet et avec laquelle le médicament est aspiré une fois dilué et mélangé et une vanne à trois voies (4), **caractérisé en ce que** le sachet de mélange (2) pour le concassage et la dispersion du médicament solide dans de l'eau est pourvu d'une première ouverture (2a) appropriée en tant qu'entrée pour l'insertion dans celle-ci du comprimé qui constitue le médicament et d'une deuxième ouverture (2b) pour le passage de fluide et **en ce que** ladite seringue (3) est une seringue entérale et est couplée à la deuxième ouverture (2b) du sachet de mélange (2) par interposition de la vanne à trois voies (4) qui, connectée hermétiquement à ladite ouverture (2b), a : avec une connexion de type ENFIt (4a) pour la connexion de la seringue entérale (3) ; avec un tube d'extension (5) pourvu d'un connecteur (6) pour un tube au niveau de l'extrémité distale de celui-ci ; et avec un robinet mobile (4b) qui, en fonction de la position dans laquelle il est situé, ouvre le passage de fluide depuis la seringue (3) vers l'ouverture (2b) du sachet de mélange (2), pour injecter l'eau de solution et pour aspirer le mélange une fois que la dissolution a été effectuée, ou depuis la seringue (3) vers le tube (5) pour délivrer le médicament dilué directement dans la bouche du patient ou par connexion du connecteur (6) à un tube nasogastrique, sans besoin de déconnecter la seringue entérale (3).

2. DISPOSITIF DE CONCASSAGE ET DE DISSOLUTION/SUSPENSION DE MÉDICAMENTS DANGEREUX POUR ADMINISTRATION ENTÉRALE, selon la revendication 1, **dans lequel** la première ouverture (2a) du sachet de mélange (2) est une ouverture circulaire équipée d'un joint à vis hermétique et résistant à la pression (7).

3. DISPOSITIF DE CONCASSAGE ET DE DISSOLUTION/SUSPENSION DE MÉDICAMENTS DANGEREUX POUR ADMINISTRATION ENTÉRALE, selon les revendications 1 et 2, **dans lequel** les deux ouvertures (2a, 2b) du sachet de mélange (2) sont les seules ouvertures que ledit sachet de mélange (2) a et il est totalement hermétique et étanche, l'intégralité du périmètre de celui-ci étant scellé au moyen d'une soudure thermique résistante à la haute pression.

4. DISPOSITIF DE CONCASSAGE ET DE DISSOLUTION/SUSPENSION DE MÉDICAMENTS DANGEREUX POUR ADMINISTRATION ENTÉRALE, selon la revendication 3, **dans lequel** le sachet de mélange (2) est en éthylène-acétate de vinyle ou en polyléfine et a une capacité d'environ 100 ml.

5. DISPOSITIF DE CONCASSAGE ET DE DISSOLUTION/SUSPENSION DE MÉDICAMENTS DANGEREUX POUR ADMINISTRATION ENTÉRALE, selon la revendication 3 ou 4, **dans lequel** les deux ouvertures (2a, 2b) du sachet (2) sont agencées sur le même côté de celui-ci.

6. DISPOSITIF DE CONCASSAGE ET DE DISSOLUTION/SUSPENSION DE MÉDICAMENTS DANGEREUX POUR ADMINISTRATION ENTÉRALE, selon la revendication 3 ou 4, **dans lequel** les deux ouvertures (2a, 2b) du sachet (2) sont sur des côtés opposés de celui-ci.

7. DISPOSITIF DE CONCASSAGE ET DE DISSOLUTION/SUSPENSION DE MÉDICAMENTS DANGEREUX POUR ADMINISTRATION ENTÉRALE selon l'une quelconque des revendications précédentes, en ce qu'une deuxième vanne à trois voies (8) est montée sur le connecteur (6) agencé au niveau de l'extrémité du tube (5), dans lequel une deuxième seringue (9) est connectable sur l'une des entrées, tandis qu'une deuxième section de tubulure (10) est connectable sur l'autre des sorties de cette vanne à trois voies (8), et au niveau de l'extrémité de laquelle se trouve un connecteur (11).

8. DISPOSITIF DE CONCASSAGE ET DE DISSOLUTION/SUSPENSION DE MÉDICAMENTS DANGEREUX POUR ADMINISTRATION ENTÉRALE sur le connecteur (6) de la première section de tubulure (5) ou sur le connecteur (11) de la deuxième section de tubulure (10) est connectable soit un bioconnecteur (12) pourvu de sections filetées « enfit » qui ne sont pas compatibles avec des cathéters intraveineux, soit des tubes nasogastriques (3).
